(19) Europäisches Patentamt
European Patent Office
Office européen des brevets

(11) **EP 4 295 786 A1**

(12) **EUROPEAN PATENT APPLICATION**

(43) Date of publication:
**27.12.2023 Bulletin 2023/52**

(21) Application number: **22180903.1**

(22) Date of filing: **24.06.2022**

(51) International Patent Classification (IPC):
**A61B 17/12** (2006.01)    **A61B 17/00** (2006.01)

(52) Cooperative Patent Classification (CPC):
**A61B 17/12; A61B 5/026;** A61B 2017/00017;
A61B 2017/00022; A61B 2017/00221;
A61B 2017/00398; A61B 2017/00876

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(71) Applicant: **Fondation EspeRare
1202 Geneva (CH)**

(72) Inventors:
• **Kant Mareda, Caroline
1253 Vandoeuvres (CH)**

• **Porte Thomé, Florence
69110 Sainte-Foy-lès-Lyon (FR)**
• **Coeudevez, Pascal
2300 La Chaux-de-Fonds (CH)**
• **Bovey, Dominique
1613 Maracon (CH)**

(74) Representative: **Cabinet Laurent & Charras
Le Contemporain
50 Chemin de la Bruyère
69574 Dardilly Cedex (FR)**

(54) **IMPLANTABLE MEDICAL DEVICE FOR CONTROLLING THE FLOW IN A BODY VESSEL**

(57)    The invention relates to a device (100) for controlling the flow in a body vessel (82) comprising a first housing (20) destined to be implanted around a body vessel (82), said housing (20) comprising means for applying partial occlusion to a body vessel (82), to regulate the flow through said body vessel (82), means for controlling the degree of occlusion applied on said vessel (82), and a second housing (30) connected to the first housing (20) through transmission means (40), said second housing (30) including an internal antenna, said internal antenna being destined to receive signals for controlling said means for applying partial occlusion. The invention is characterized in that said second housing (30) is destined to be implanted subcutaneously and said means for controlling the degree of occlusion are located in said second housing (30).

**Figure 2**

EP 4 295 786 A1

## Description

## Field of the invention:

The invention relates to the field of flow control and more precisely to a device for controlling the flow in a body vessel. The device of the invention has numerous therapeutic applications including the banding of the pulmonary artery for patients, including premature neonates with low birthweight suffering from congenital heart defects. The invention may also find an application in banding the portal vessels after a liver transplant. The invention is also useful to induce a model of right or left heart failure in large animals.

## Background:

Congenital heart diseases (CHD) are defined as structural abnormalities of the heart and/or great vessels, that is present at birth. CHDs affects 1 in 100 newborns each year. In 5% of cases, the severity of CHD is life threatening and is responsible for the largest proportion of mortality caused by birth defects. Severe cases are treated by cardiac surgery at birth. When this is not possible, because the health status of the newborn baby does not allow open-heart surgery at birth, Pulmonary Artery Banding (PAB) is used as a palliative technique, until the reparative surgery is possible, to control blood flow in the Pulmonary Artery (PA) to avoid subsequent development of life-threatening and irreversible complications. Traditionally, PA banding are performed using a Gore-Tex® band which contriscts the PA. With such technic, re-operations are often required to adjust the occlusion of the banding when the patient grows up or the disease progresses and worsens. This has dramatic impacts on the costs of illness and on the patients' quality of life and causes prolonged hospital stays. Moreover, conventional PAB with Gore-Tex® bands may lead to unwanted scarring and fibrosis, which often requires reconstruction of the PA at the time of banding removal. Also, anesthetic agents used for the induction of general anesthesia often perturb hemodynamic, such that it is challenging to precisely regulate the degree of occlusion to achieve the desired pressure gradient. Therefore, there is a need for a remotely adjustable device to avoid re-operations and improve the precision of the banding. FloWatch® is a one size device which was commercialized for congenital heart diseases from 2003 to 2012. During the commercialization, the device was successfully implanted in hundreds of patients.

FloWatch® is a wireless, battery free implantable device consisting of a mechanical actuator controlled by a micromotor. It was implanted through a simple surgery procedure either sternotomy or thoracotomy. The device was placed around the PA or the PA trunk. After implantation, and once the patient was awake and free from anesthetic drug, it was possible to adjust the degree of occlusion precisely and remotely under echocardiography, in order to achieve the desired pressure gradient without re-operating the patient. To receive commands to control the pressure exerted on the artery, the device included an antenna directly in the device's housing. Such adjustments were performed several times during the implantation lifetime, which was set to 6 weeks for FloWatch® PAB.

However, the FloWatch® device had a very limited number of therapeutic applications, which led to the discontinuation of the commercialization of the device because of key technical challenges.

Firstly, radiofrequency telemetry, used to transmit the control signal to the means for controlling the degree of occlusion, requires large amounts of power and results in lowefficiency transmission through biological tissue and some challenge with the FloWatch® was about the energy transmission which was suboptimal: the maximum distance between the external command and the device antenna had to be less than 4 cm and the angle less than 25° for it to function effectively. Hence, it was not possible to implant it in patients with body weight greater than 6 kg or in large animals or even on more profound arteries.

Moreover, the FloWatch® device is too bulky to be implanted in the thoracic chest of premature babies and neonates. The bulkiness of the device prevented its use in premature babies with a bodyweight under 2,5 kg. In some cases, the device's weight could also lead to kinking of the PA.

Also, the device is not convenient as it can only open from one side. Thus, during implantation into the patient's body, depending on the local environment, the clinician will not be able to close the device around the artery because he is hindered by the presence of obstacles. Due to the geometry and one length of the clasp, FloWatch® PAB was suited only for PAB banding in babies with a PA diameter of 10 mm.

Lastly, FloWatch® PAB was a one size device designed to band vessels with a diameter of 10 mm.

FloWatch® PAB is inspired from document EP 1072282. Figure 1 of document EP 1072282 shows a flow control device 120 implanted nearby a blood vessel 82. The device 120 comprises a housing 152. Contrary to the FloWatch® device, document EP 1072282 also envisioned an antenna 132 located outside the housing, so that it is not screened by the housing. Yet this device was never manufactured nor commercialized for congenital heart diseases or other indications. The antenna **132** receives information from a control device **122** via a second antenna **130**. The antennae **130, 132** are electromagnetically coupled to each other, on opposite sides of the patient's skin.

A receiver **134** is connected to antenna **132** with a wire designed for the transmission of radio signals. Said receiver **134** converts radio frequency energy received by said antenna **132** into electrical energy to power a motor **136**. When provided with electrical power, the motor **136** can be used to rotate a drive shaft **138**. A rotary to linear transmission **140** converts the rotation of the drive shaft **38** into linear motion of an actuator comprising members **142, 144** and **146.**

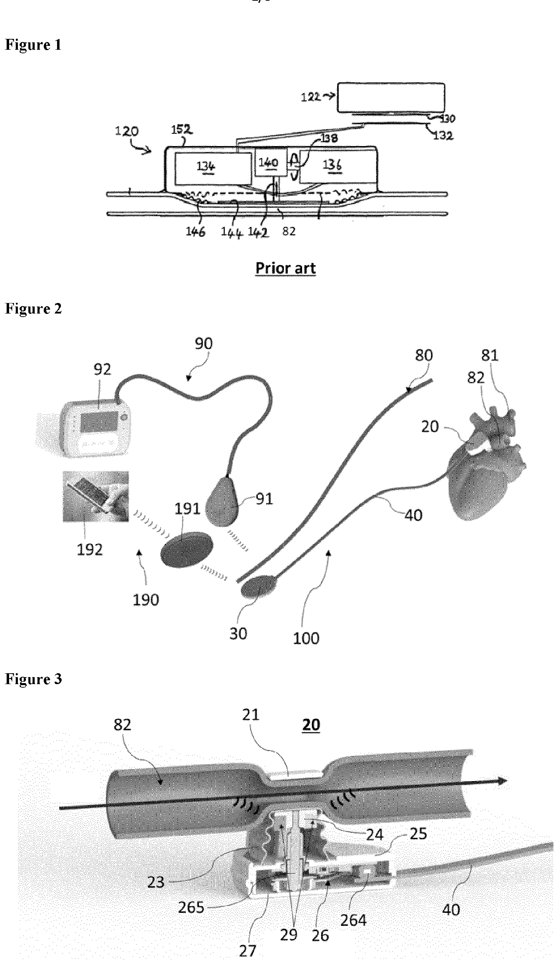

Even though the device from Figure 1 shows an antenna that is located outside the housing so that it is not screened by the housing, this does not solve the issue of distance between the external antenna and the internal antenna. Thus, the device from Figure 1 may be more flexible than the FloWatch®, but it still needs to be implanted close to the skin surface for it to function efficiently. Moreover, as the antenna is located outside the housing, it is not fixed and can move freely inside the body. This represents safety issues to the subject. The antenna is powered by electricity and the wire might get damaged by interference with the inner body electrical and/or chemical/osmotic and/or mechanical reactions, thus resulting in localized heating to surrounding tissues and/or unintended electrocution.

The invention seeks to overcome the issues described above in order to broaden the therapeutic indications, the eligible population as well as the safety and efficacy of the device.

## Summary of the invention:

In order to solve these issues, an improved miniaturized implantable medical device was developed. The device has a deported second housing including an internal antenna. This housing can be implanted subcutaneously to improve transmission of a control signal coming from outside the subject's body. Thus, the invention guarantees a perfect connection between the external control device and the second housing. This connection allows to simultaneously transfer energy and control signals to the device of the invention.

According to the present invention there is provided an implantable medical device for controlling the flow in a body vessel. It comprises:

- a first housing destined to be implanted around a body vessel, said housing comprising means for applying partial occlusion to a body vessel, to regulate the flow through said body vessel,

- means for controlling the degree of occlusion applied on said vessel, and

- a second housing connected to the first housing through transmission means, said housing including an internal antenna, said internal antenna being destined to receive control signals for controlling said means for applying partial occlusion.

The invention is characterized in that said second housing is destined to be implanted subcutaneously, said means for controlling the degree of occlusion of the body vessel being located in said second housing.

According to the invention, a "body vessel" may be any tract, tube, duct, or vessel of the body that can transport a liquid. For instance, a body vessel may be a urinary tract, the biliary tract, an intestine, or the esophagus and even an artificial shunt.

In the prior art, the internal antenna was located outside the first housing to avoid being screened by it and thus preventing transmission of the control signals. However, if the device was implanted in patients weighing more than 6 kg, the internal antenna was too deep to receive the info from the external antenna. Moreover, this solution was unsafe because the antenna is not fixed and can move freely inside the subject's body. Such movement of the antenna may lead to the migration of the first housing and or damage of the antenna.

The invention solves this issue by deporting the internal antenna in a second housing that is implanted and fixed by a surgery stitch subcutaneously.

By subcutaneously, it is meant that the second housing may be implanted from 0.5 to 5 cm under the skin, advantageously from 1 to 3 cm.

Therefore, the antenna and wire within the second housing are fixed inside the body, and they cannot move freely. Advantageously, the second housing may be sutured to the rectus sheath. The device from the invention is thus safer for the implanted patients.

By deporting the means for controlling the degree of occlusion in the second housing, the means for controlling the degree of occlusion and the internal antenna get much closer. Hence, the information travels on a shorter distance and it is not distorted.

For instance, said transmission means is an electric wire with a protective sleeve made of Liquid Crystal Polymer.

Additionally, by deporting the means for controlling the degree of occlusion in the second housing, the first housing was miniaturized by at least 30% in size and weight. Moreover, its shape and geometry were specifically designed to match the anatomy of a body vessel. As such, the device from the invention can be implanted without any risks of causing a kink to the body vessel. The risks of migration of the device along the body vessel are also reduced due to the newly improved geometry of the second housing. Accordingly, the second housing has advantageously a polygonal shape such as a pentagonal or hexagonal shape. Moreover, the surgeon's work is easier because of the miniaturization, weight, and specific geometry.

Also, there is no restriction with the positioning of the implanted medical device, as the deported internal antenna allows an effective transfer of energy and information between an external control device and the first housing.

More specifically, the means for controlling the degree of occlusion comprise an electronic card connected to said internal antenna, said electronic card comprising a field-programmable firmware.

According to the invention, a field-programmable firmware may be programmed before implanting the device.

Said device may also include an external control device comprising an external antenna, the internal antenna being electromagnetically coupled to the external antenna, said internal antenna receiving control signals from the external control device through the external antenna, said internal antenna transmitting said control signals to the field-programmable firmware.

Advantageously, said external control device includes a first magnet and said second housing includes a second magnet, both magnets being attracted to each other to better align the external antenna with the internal antenna.

In some embodiments, the device may be only powered by the energy transmission between both antennae. As such, it is very important to optimize the energy transmission, for instance by using such magnets.

In other words, a user may approach the external antenna of the external control device toward the second housing that is implanted subcutaneously. When the external antenna is close enough to the second housing, the magnets start to attract each other until they get in contact. This attraction guides the positioning of both external and internal antennae to improve signal transmission between the two antennae.

Moreover, the means for applying partial occlusion of the device comprise:

- a clasp destined to surround the body vessel at least partially, and

- a motor assembly including a compression piston for releasably press on said body vessel.

Advantageously, the motor assembly includes a micromotor.

Therefore, the clasp partially surrounds the body vessel on one side and the compression piston presses on the wall of the body vessel on the other side. When the device is placed around the body vessel, said body vessel changes shape to adopt a banana shape.

The banana shape doesn't impact the flow rate as it is necessary to have an occlusion of more than 80% of the body vessel to have an impact on flow rate. Thus, the flow rate can only be controlled by moving the compression piston.

However, the banana shape allows to keep the perimeter of the body vessel while reducing its diameter. Banding devices using Gore-Tex® bands of the prior art were not able to obtain the same results. The body vessel was non-homogeneously constricted, thus causing turbulences to appear inside the body vessel. Turbulences can be harmful as they can result in thrombosis. Therefore, the device of the invention is safer and more reliable because there is no risk of turbulences as the perimeter of the banded vessel is not modified by the banding and it also allows the artery wall to grow and to be protected from necrosis. Compared to existing technologies, the device also allows a more precise control on the occlusion as the compression piston can be controlled with a precision of about 0.1 mm.

Said clasp may be releasably fastened on attachment means present on the upper side of said first housing. The releasability is convenient as it allows to re-open the device in order to extract it from the body once it is no longer useful. The releasability may also be used to change the clasp size for a smaller or larger size if a desired flow rate cannot be reached.

In use, the surgeon may position the first housing on one side of the body vessel and the clasp on the other side. A cooperation of the attachment means with the clasp allows to secure it around the body vessel.

Advantageously, a range of differently sized devices is produced to better match the diameter of the body vessel to band. As such, it is possible to pre-select a device with predetermined dimensions according to the diameter of the body vessels to be banded and its localization in the body. For instance, the device may be applied to babies' arteries or shunt as little as 4 mm of diameter. It may also be used to band vessels in adult patients or in large animals of various sizes. More precisely, the dimensions of the first housing as well as the size of the clasp may vary between two devices of different size.

Advantageously, the clasp may open along a first direction or a second direction depending on the way it was fastened on said attachment means.

This feature facilitates the surgery. In particular, for the case of pulmonary artery banding in premature babies, depending on the anatomy, access to the device may be hindered by the presence of organs or tissues or other body vessels. Thus, if the surgeon has a choice on the opening direction of the clasp, he may be able to increase the number of accessible positions around a given body vessel, thus improving the success of the surgery and the number of eligible patients to this surgery procedure.

The clasp may have a symmetrical shape to allow the clasp fastening in two orientations on said attachment means, each orientation corresponding to a chosen opening direction of said clasp.

Additionally, the first and/or second housing may include fixation means for attaching said first and/or second housing to the surrounding tissues. The surgeon may therefore add a stitch close to the implanted first and/or second housing, to maintain the housing in place. For instance, the fixation means may prevent the reopening of the clasp after implantation. A ring might also be added on the implant, next to the wire exit, in order to stich it in place. The stitch will be used to secure the antenna, most likely by suturing it to the rectus sheath.

Preferably, the first housing may further comprise a radar for measuring and recording the pressure gradient to determine the degree of occlusion applied by said compression piston. This allows to get feedback on the compression piston position and to verify that the desired degree of occlusion was obtained. If the target is not reached, it is possible to move again the compression piston to apply more/less pressure. This feedback loop on the piston position allows to reduce the risks of overpressure in the body vessel. Advantageously, said radar is a radiofrequency radar.

According to another aspect, the invention relates to an animal model comprising a living non-human animal implanted with the device of the invention. Said animal may be a pig, a dog, a cow, a goat or even a sheep.

Moreover, the invention also relates to the use of the above living non-human animal as animal model of cardiac failure.

The animal model may be used to study the effect of a disease on the animal and to test the effect of potential drugs.

**Brief description of the Drawings:**

The Applicant has developed an improved device that answers the issues raised previously.

Figure 1 is a schematic cross-sectional view of a banding apparatus from prior art,

Figure 2 is a perspective view of the device according to a first embodiment of the invention,

Figure 3 is a cross-sectional view of the first housing of the device from figure 2,

Figure 4 is an exploded view of the device from figure 2,

Figure 5 is a perspective side view of the motor assembly of the device from figure 2,

Figure 6 is a perspective top view of the motor assembly of the device from figure 2,

Figure 7 is a cross-sectional view of the second housing of the device from figure 2, and

Figure 8 is a comparative view of the FloWatch® apparatus from prior art and the device from figure 2 with comparative dimensions.

**Detailed description of the realization modes:**

As illustrated in figure 2, the device **100** comprises a first housing **20** and a second housing **30**. Both housings **20, 30** are connected to one another with a transmission wire **40.**

The device **100** may be wholly implanted inside a subject. As such, in figure 2, the subject's skin is represented by a line **80**. It is the interface between the inside of the subject and the outside world.

The first housing **20** may be implanted around an artery **82** of the heart **81,** such as the pulmonary artery or the aorta. In other realization modes, the device **100** may be installed around any vessel of the body such as the portal vein leading to the liver, a urinary duct or even around an artificial shunt.

A body vessel according to the invention may have a diameter comprised between 5 to 40 mm, with an average size of 12 mm.

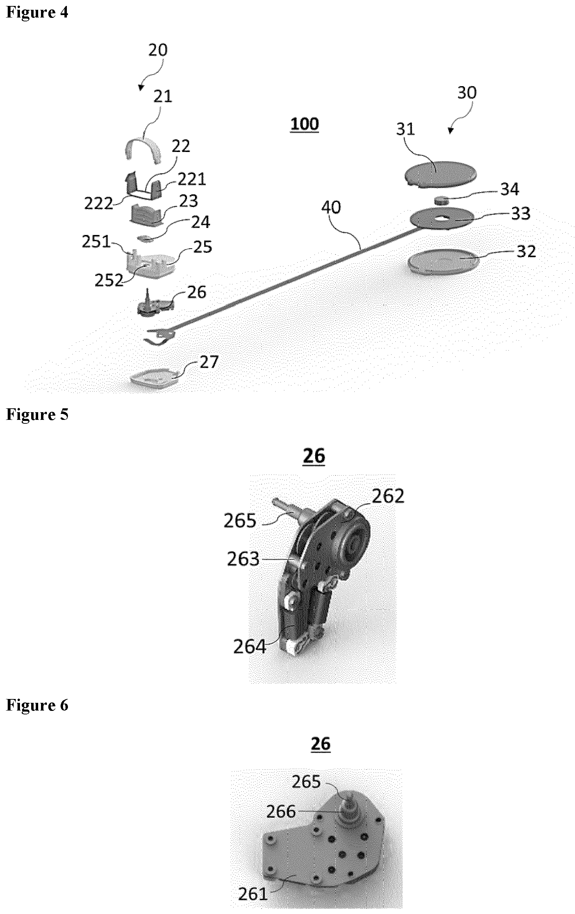

As illustrated in figures 3 and 4, the first housing **20** has a hexagonal or pentagonal shape. It comprises an upper part **25** and a lower part **27**. Both upper and lower parts **25, 27** cooperate to form an inner cavity destined to receive a motor assembly **26**. The inner cavity is hermetic to fluids in order to protect said motor assembly **26** from humidity and erosion. Hermeticity may be archived using a gasket. Both upper and lower parts **25, 27** may be made of a biocompatible material such as ceramic, titanium or a titanium alloy in order to avoid body rejection reactions. As an alternative, both upper and lower parts **25, 27** of the housing may be 3D printed or obtained with plastic injection using PolyEther Ether Ketone (PEEK) material.

As illustrated in figures 5 and 6, the motor assembly **26** comprises a metallic skeleton with an upper plate **261** and a lower plate **262** kept together with a set of screws **263.** The motor assembly **26** also includes a miniature motor, not shown in the figures, usually used in watches. The motor may be 1 to 5 mm thick and 13 to 17 mm long. The motor is positioned between the upper and lower plates **261, 262.** The motor is connected to two electrical contacts **264** that provide the motor with electrical power. In use, the electrical contacts **264** are in contact with the transmission wire **40,** as illustrated in figure 3.

When provided with electrical power, the motor can be used to rotate a drive shaft, not shown in the figures. A rotary to linear transmission converts the rotation of the drive shaft into linear motion of a compression piston **265**. The compression piston **265** has a tubular shape with a diameter comprised between 0.1 to 0.5 cm. The length of the compression piston **265** may vary between 0.5 and 1.5 cm.

The upper plate **261** comprises an opening **266** through which said compression piston **265** may extend.

Similarly, the upper part **25** of said first housing **20** has an opening **252**. The motor assembly **26** is positioned inside said inner cavity of the first housing **20** so that the compression piston **265** may extend through the opening **252** toward the body vessel **82.**

As illustrated in figures 3 and 4, the top of the compression piston **265** is fitted with a pressure piece **24** whose function is to apply the pressure on said body vessel **82**. As such, the pressure piece **24** may have a parallelepiped or cylindrical shape of 0.5 to 1 cm long and 0.2 to 0.5 cm wide and 0.2 to 0.5 cm high. Preferably, the upper surface of the pressure piece **24** designed to come in contact with the body vessel **82** has a more rounded shape to avoid sharp edges to cause damage to the membrane covering it **23** and to the body vessel **82**. Preferably, the pressure piece **24** may take the general shape of a banana.

The pressure piece **24** and the exposed portion of the compression piston **265** are covered with a membrane **23**. The membrane **23** has a concertina shaped arrangement with collapsible gussets capable of extending when the length of the compression piston **265** increases and to collapse back when the length of the compression piston **265** decreases.

Said membrane **23** is sealed to the upper part **25** of the first housing **20** using a framing piece **22**. The framing piece **22** comprises a rectangular-shaped base **222** and two lateral arms **221** extending on each side of said base **222**. The framing piece base **222** is fixed on the upper part **25** of the first housing **20**. The membrane **23** is therefore sandwiched between said framing piece base **222** and said upper part **25**. To that end, the upper part **25** may comprise reception sites where the framing piece base **222** can be glued, clipped, or screwed. In use, the two lateral arms **221** act as a guide to the movements of the membrane **23**. The lateral arms **221** may be spaced from each other with a length comprised between 0.5 to 3 cm.

A clasp **21** is added on top to frame the body vessel **82**. Preferably, the clasp **21** has a hemiellipsoidal shape.

Traditionally, an ellipse is defined by its minor axis and major axis. The clasp **21** may have a minor axis with a length comprised between 0.2 to 1.5 cm and a semi-major axis with a length comprised between 0.2 to 3 cm. Moreover, the width of the clasp **21** may be comprised between 0.2 to 1 cm, advantageously 4 mm.

A range of differently sized devices **100** may be produced. Each device **100** having a predetermined size of clasp **21** and a predetermined size of first housing **20**. One may choose from the available devices **100** of different sizes to find the best fit for a given body vessels **82**. For instance, for bigger body vessels **82,** the first housing **20** may be larger to better circle the body vessel **82** and the corresponding clasp **21** may have a bigger major axis.

As shown in figure 4, the clasp **21** may be releasably fastened to the upper part **25**. To this end, the upper part **25** may comprise attachment means **251**. The clasp **21** is clipped to these attachment means **251** so that one end of the clasp **21** may rotate around an axis to allow the other end of the clasp **21** to open.

For this purpose, the clasp **21** has, on each end of the hemi-ellipsoid, a hook destined to cooperate with the attachment means **251** and holes to fit a pin.

The attachment means **251** are symmetrical and comprise on each side two tubular portions facing each other adapted to receive the pin, and deformable protrusions configured to cooperate with a hook.

Thus, to fix the clasp **21** on the upper part **25,** the holes present on one side of the clasp **21** are aligned with the tubular portion on one side of the upper portion **25** and the pin is inserted through both the holes and tubular portions to maintain them together and allow a rotation around the pin.

The hook present on the other end of the clasp **21** may then cooperate with the deformable protrusions to close the clasp **21.**

A stitch may be added around the hook to avoid the reopening of the clasp **21** during its stay inside the body.

The upper part **25** and the attachment means **251** may be a monobloc unit that may be obtained using 3D printing or plastic injection.

The clasp **21** is symmetrical and it is possible to fix the clasp **21** on the attachment means **251** in two directions. Thus, the clasp **21** may be opened from the left or from the right depending on how it was positioned.

In use, the first housing **20** may be positioned on one side of the body vessel **82,** while the clasp **21** is positioned on the other side. The clasp **21** is engaged with the attachment means **251** to surround the body vessel **82**. If the surgeon needs to change the position of the first housing **20** or to simply remove it, he may exert pressure on the deformable protrusions to release the clasp **21** and allow its opening.

The clasp **21** opening direction is chosen depending on the surroundings of the body vessel **82.** For instance, if an organ or tissues hinders access to the device on the left, the surgeon may choose to close the clasp **21** on the right side to improve accessibility.

The clasp **21,** framing piece **22**, membrane **23** and intermediate piece **28** may be made of a biocompatible material such as PEEK.

In use, when the body vessel **82** is compressed by the pressure piece **24,** it tends to deform toward the sides and the top. The clasp **21** prevents this kind of deformation and guarantees that the diameter of said body vessel **82** is effectively reduced.

As illustrated on figures 3, 4 and 7, the second housing **30** may be implanted subcutaneously. For instance, the second housing **30** may be implanted from 0.5 to 5 cm under the skin depending on the applications.

The second housing **30** comprises an upper portion **31** and a lower portion **32**. Both upper and lower potions **31, 32** cooperate to form an inner cavity destined to receive an electronic card **33**. The inner cavity is hermetic to fluids in order to protect said electronic card **33**. Hermeticity may be archived using a gasket. Both upper and lower potions **31, 32** may be made of a biocompatible material such as ceramic, in order to avoid rejection reactions. As an alternative, both upper and lower potions **31, 32** of the housing **30** may be 3D printed or obtained with plastic injection using Polyether ether ketone (PEEK) material.

The second housing **30** may be secured subcutaneously with stitches, preferably localized on the rectus sheath. A ring may be positioned on the second housing **30,** close to the cable, for that purpose.

Advantageously, the electronic card **33** includes the electronics for controlling the degree of occlusion of the body vessel **82**. As such, the electronic card **33** includes firmware. The firmware may comprise a non-volatile memory integrated circuit such as ROM, EPROM, EEPROM or Flash memory to store the control sequence of the compression piston **265** position.

The firmware is field-programmable, meaning that it may be programmed *in situ* without opening the subject's body to access to the device.

The electronic card **33** comprises an internal radiofrequency antenna that may be directly printed on the electronic card **33** and that receives information from a control device **90, 190** via a second antenna. The antennae are electromagnetically coupled to each other, on opposite sides of the patient's skin. For this purpose, a magnet **34** may be included on the electronic card **33**. The magnet **34** is used to position the external antenna, which is also equipped with a magnet. Both magnets **34** will be attracted to each other, which allows a better alignment of the second housing with the external control device and a better transmission of information.

The electronic card **33** also includes a receiver connected to the internal antenna Said receiver converts radio frequency energy received by said antenna into an electric signal.

The electric signal is transmitted to the motor assembly **26** with transmission means **40**. Preferably, the transmission means is an electric wire **40** that is biocompatible and connects both housings **20, 30**. For instance, the electric wire **40** may pass through an intercostal space to link both housings **20, 30**.

The biocompatible electric wire **40** is connected on one side to the electronic card **33** and on the other side to the electrical contacts **264**. It is preferably made with a Liquid Crystal Polymers (LCP) protective sleeve, a very inert material, capable of resisting stress cracking when confronted with chemicals at high temperatures. The tensile strength of LCPs is 12,000-32,000 psi with a temperature resistance up to 260°C. The various properties of LCPs make them useful in several applications that require high strength. The low dielectric constants of the polymer make LCP great for electronics with high frequency. LCP is also biocompatible according to ISO 10993-5 standard.

The electric wire **40** length is comprised between 10 and 30 cm. Preferably, the wire **40** has a flat section and it can be oriented in any direction with less risk of kinks. The wire **40** is preferably connected at the opposite end of the clasp **21** position on the second housing **20**.

As illustrated on figure 2, the device **100** may be controlled using a control device **90, 190**. The control device may include a smartphone **192** with a compatible smartphone application. The smartphone **192** may send control signals.

The smartphone **192** may be connected to an independent module **191,** which is also part of the control device **190** and is responsible for transforming and transferring commands from the smartphone **192** to the device **100** using a radiofrequency connection.

As an alternative, the control device **90** comprises a first housing **92,** connected to a second housing **91**. The second housing **91** transfers commands from the first housing **92** to the device **100** using a radiofrequency connection.

The independent module **191** or second housing **91** include a processor control and software management means. They also include a radiofrequency-emitting antenna, advantageously a memory to store data collected by the device **100** or even a battery.

The control device **90, 190** has a user interface allowing a user to set the pressure exerted on the body vessel **82**. The control device **90, 190** may also include a feature to parameter a pressure pattern over time, to simulate the evolution of disease for instance.

The control device **90, 190** may also be configured to drive the device **100** to perform programmed cycles. More precisely, the program embedded in the control device **90, 190** may be programmed to control the opening and closing of the device **100** to execute identical or variable cycles over a given period of time, without having to use the manual control.

Advantageously, the device **100** is autonomous and only powered to perform opening and closing cycles once the magnets **34** are aligned so that the external control device **90, 190** is in close contact with the second housing **30**.

In both cases, the communication between both antennas is wireless. The external second antenna supplies enough radiofrequency energy to power the receiver and allow the implanted device **100** to work without requiring a battery.

Advantageously, in another embodiment, the first housing **20** may include means for measuring the blood pressure inside said body vessel **82**.

As such, the first housing **20** may include at least one sensor **29**. Information from the sensor(s) **29** can then be fed-back to the control device **90, 190** either via the second housing **91** or via the module **191** so that the control device **90, 190** may control the pressure exerted on the body vessel **82** more precisely.

For instance, as illustrated in figure 3, the first housing **20** may include two sensors **29** located on the pressure piece **24**. The sensors **29** are preferably located close to the body vessel **82** in order to reduce noise occurrence.

A potential technique to measure a gradient of pressure is to measure the flow velocity and extract the pressure using the Bernoulli principle.

If K is the kinetic energy of blood cells and P, the pressure energy calculated on a cross-sectional plane of the body vessel **82**, the Bernoulli equation state that between two cross-sectional planes of the body vessel **82**:

$$K_1 + P_1 = K_2 + P_2$$

Assuming that two separate velocity sensors are mounted around the body vessel **82,** each sensor can measure the blood flow velocity that passes through plane 1 and plane 2. Starting from the Bernoulli equation and by rewriting the kinetic energy as a function of velocity, the pressure difference between the planes may be approximated by:

$$\Delta P = 4(V_1{}^2 - V_2{}^2)$$

Where $V_1$ and $V_2$ are the blood flow velocity measured by the sensors at a specific plane inside the artery.

In another embodiment, the blood pressure may be measured using a radar.

A radar system consists of a transmitter producing electromagnetic waves, a transmitting antenna, a receiving antenna and a receiver and processor to determine properties of the object. The electromagnetic wave sent by the transmitter is reflected on the target object and returns to the receiver, giving information about the object's location and speed.

Preferably, the radar is a radiofrequency radar. Indeed, radiofrequencies radiation are nonionizing and harmless for the body at low power levels. Alternatively, the radar may be based on ultrasonic or laser technology.

For instance, an in-body piezoelectric sensor can be used to measure the arterial flow pressure using ultrasonic waves. Preferably, the sensor is made of a polymer-based piezoelectric material. Infrared Photoplethysmography (PPG) is another possibility to measure the flow pressure inside the artery.

For example, the radars may be an ultra-wideband radar based on CMOS technology. The center frequency may be comprised between 7 and 7.5 GHz, preferably of 7.25 GHz, with a bandwidth comprised between 2 and 3 GHz, preferably of 2.5 GHz. Such radar shows a consistency of 82% with electrocardiography corresponding measurements.

For instance, a 60 GHz bistatic pulsed coherent radar may be used. This kind of sensor transmits coherent pulses with the repetition rate of 13 MHz. The sensor dimensions are very limited, e.g about 5mm×5mm and its operating temperature is between -40° to 85°C. The power supply voltage is 1.8 V and the power consumptions measures in mW.

In use, a radiofrequency signal is transmitted through the artery **82** and reflects on moving blood particles. To determine the speed of said blood particles, the Doppler effect is used. The Doppler effect is based on the change in frequency observed when the electromagnetic wave reflects on a moving target. More precisely, the measured frequency reflected by a moving target, with a velocity of v, shifts proportionally to the target speed. Hence, if the radar transmits radiofrequency waves with a wavelength of $\lambda$, the frequency shift $f_D$ is obtained by the following formula:

$$f_D = 2v / \lambda$$

However, blood particles can reach very high speeds. To follow this evolution, it is necessary to have a high frame rate. Therefore, the radar may advantageously transmit N consecutive pulses with a repetition interval $T_{PRI}$. The N received signals are collected and stored in a 2D array. Afterwards, a Fourier transform is used to obtain the IQ signals spectrum.

The IQ signals are two amplitude-modulated signals: the in-phase I signal and the quadrature Q signal. The two signals are sinusoids that have the same frequency and are 90° out of phase. By convention, the I signal is a cosine waveform, and the Q signal is a sine waveform. The modulation is obtained using a signal that is generated by a local oscillator.

However, most of the information contained in the IQ signals corresponds to the tissue signal, which generally has a lower frequency and a much greater amplitude than that of blood signals. Thus, the signal contains a lot of noise. In order to retrieve only the information on blood particles displacement, it is possible to filter the signals using a low pass filter and amplify them using a Low Noise Amplifier LNA.

Afterward, the speed may be retrieved by converting the IQ signals spectrum into speed.

Alternatively, the first housing **20** may include other sensors such as oxygen level sensors.

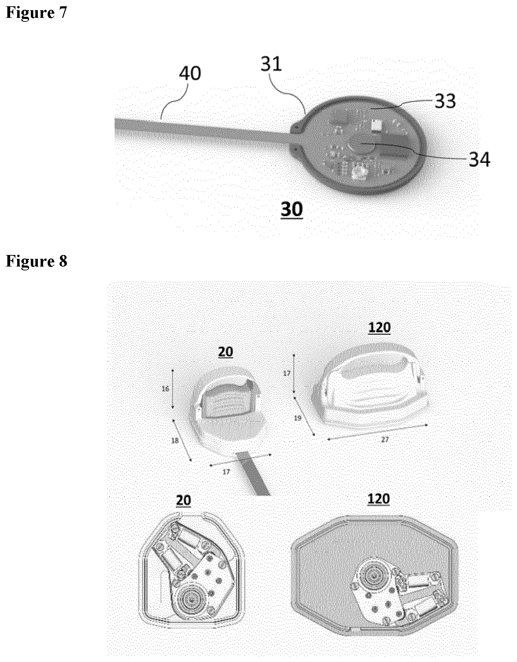

As illustrated in figure 8, the first housing **20** has a size of 18 mm long, 17 mm width and 16 mm high while the FloWatch® device **120** has a size of 19 mm long, 27 mm large and 17 mm high. A 30% miniaturization was obtained by removing the electronic card from the first housing and repositioning the motor assembly inside the first housing **20.**

The device **100** of the invention has many applications. Examples are described below, but they should not be interpreted as limitative.

In a first application, the device **100** may be used as a palliative technique to control blood flow in the Pulmonary Artery for newborns suffering from a Congenital Heart Disease. Indeed, Pulmonary arterial hypertension is a frequent complication of CHD, particularly in patients with cardiac left-to-right shunts. Persistent exposure of the pulmonary vasculature to increased blood flow and pressure may result in vascular remodeling and dysfunction and Pulmonary arterial hypertension ultimately..

In order to avoid vascular remodeling, the device may be implanted around the Pulmonary Artery to reduce excessive pulmonary blood flow and protect the pulmonary vasculature from hypertrophy and irreversible pulmonary hypertension.

In a second application, the device **100** may be used for the treatment of small-for-size syndrome after a partial liver transplant. Indeed, a liver may be separated in several grafts and still function properly once transplanted. The partial liver is even capable of reconstituting itself into a full-fledged liver after being transplanted.

However, at the time of implantation, the graft size may be too small for the blood flow provided by the portal vein. This portal hypertension leads to stress on the liver cells, which results in subsequent processes of hepatocellular damage and cell death. Ultimately, the graft may be rejected.

In order to avoid this syndrome, the device **100** may be used to regulate the flow inside the portal vein depending on the liver size. The flow may be adjusted remotely as the liver grows back to its normal size. Once the liver has a normal size, the device **100** may be removed.

In a third application, the device **100** may be used in large animals to develop reproducible animal models of a pathology triggered by abnormal pressure or gradient in a body vessel. There are no therapies that directly target and support the failing right heart and translation from therapies that improve left heart failure have been unsuccessful due to the lack of predictability of animal models to study that symptom. A way to achieve this is to define a protocol to induce reproducible right or left heart failure in animal models. The study of such animal model may bring more reliable data allowing a better understanding of the disease and the development of new treatments.

For instance, the animal may be a pig, a dog, a cow, a goat or a sheep.

The device **100** may be used to band the pulmonary artery of the animal. The device is placed around the artery to achieve a right ventricular pressure of about 70% to 80% of the maximal pressure. The tightening may be kept intermittently or continuously depending on the type of desired heart failure. For example, it may be kept for 20 to 30 minutes before being released for about 20 to 30 minutes and then the process is repeated. The device may be kept for a few weeks and then the hemodynamic data is studied, or the animal is sacrificed to analyze the changes induced in the animal body. It is also possible to analyze if and how fast the animal recovers back to its original state.

Alternatively, any other artery may be banded by the device.

These disease animal model may also be used to test the effect of potential new therapeutic drugs.

To conclude, the device of the invention is very flexible with its reduced size and new clasps and geometry and improved energy transmission. It has a very large variety of applications, and it is easier to implant in a subject, such as for example an animal or around any type of body or artificial vessel regardless of its diameter.

**Claims**

1. Implantable medical device (100) for controlling the flow in a body vessel (82) comprising:

   - a first housing (20) destined to be implanted around said body vessel (82), said housing (20) comprising means for applying partial occlusion (21, 24, 265) to a body vessel (82), to regulate the flow through said body vessel (82),
   - means for controlling the degree of occlusion applied on said vessel (82), and
   - a second housing (30) connected to the first housing (20) through transmission means (40), said housing (30) including an internal antenna, said internal antenna being destined to receive control signals for controlling said means for applying partial occlusion (21, 24, 265), *characterized in that* said second housing (30) is destined to be implanted subcutaneously and said means for controlling the degree of occlusion (33, 90, 190) are located in said second housing (30).

2. Implantable medical device according to claim 1, *characterized in that* the means for controlling the degree of occlusion comprise an electronic card connected to said internal antenna, said electronic card (33) comprising a field-programmable firmware.

3. Implantable medical device according to claim 2, *characterized in that* said device also includes an external control device (90, 190) comprising an external antenna, the internal antenna being electromagnetically coupled to the external antenna, said internal antenna receiving control signals from the external control device (90, 190) through the external antenna, said internal antenna transmitting said control signals to the field-programmable firmware.

4. Implantable medical device according to claim 3, *characterized in that* said external control device (90, 190) includes a first magnet and said second housing (30) includes a second magnet (34), both magnets being attracted to each other to better align the control device (90, 190) with the second housing (30).

5. Implantable medical device according to claim 1, *characterized in that* said transmission means (40) is an electric wire with a protective sleeve made of Liquid Crystal Polymer.

6. Implantable medical device according to claim 1, *characterized in that* the means for applying partial occlusion comprise:

   - a clasp (21) destined to surround the body vessel (82) at least partially, and
   - a motor assembly (26) including a compression piston (265) for releasably press on said body vessel (82).

7. Implantable medical device according to claim 6, *characterized in that* said clasp (21) is releasably fastened on attachment means (251) present on the upper side of said first housing (20).

8.  Implantable medical device according to claim 7, *characterized in that* said clasp (21) may open along a first direction or a second direction depending on the way it was fastened on said attachment means (251).

9.  Implantable medical device according to claim 7, *characterized in that* said clasp (21) has a symmetrical shape to allow the clasp (21) fastening in two orientations on said attachment means (251, 281), each orientation corresponding to a chosen opening direction of said clasp (21).

10. Implantable medical device according to claim 1, *characterized in that* said first housing (30) has a polygonal shape.

11. Implantable medical device according to claim 1, *characterized in that* said first housing (20) further comprises a radar for measuring and recording the pressure gradient to determine the degree of occlusion applied by said compression piston.

12. Implantable medical device according to claim 10, *characterized in that* said radar is a radiofrequency radar.

13. An animal model comprising a living non-human animal implanted with the implantable medical device according to any of claims 1 to 11.

14. Use of the animal model according to claim 12 for creating an animal model of cardiac failure.

15. Use according to claim 13, **characterized in that** the animal is pig, a dog, a cow, a goat or a sheep.

**Figure 1**

**Prior art**

**Figure 2**

**Figure 3**

Figure 4

Figure 5

**26**

Figure 6

**26**

**Figure 7**

**Figure 8**

Europäisches
Patentamt

European
Patent Office

Office européen
des brevets

## PARTIAL EUROPEAN SEARCH REPORT

under Rule 62a and/or 63 of the European Patent Convention.
This report shall be considered, for the purposes of
subsequent proceedings, as the European search report

Application Number

EP 22 18 0903

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2010/125288 A1 (GELFAND MARK [US] ET AL) 20 May 2010 (2010-05-20) | 1-3,6,7, 10 | INV. A61B17/12 |
| Y | * paragraph [0057] - paragraph [0058] * <br> * paragraph [0063] - paragraph [0067] * <br> * paragraph [0071] * <br> * figures 1-4A * | 4,5,8,9, 11,12 | ADD. A61B17/00 |
| | ----- | | |
| Y | US 2020/187816 A1 (LEABMAN MICHAEL A [US]) 18 June 2020 (2020-06-18) <br> * column 0246 * <br> * figures 51A-C * | 4 | |
| | ----- | | |
| Y | US 2015/099959 A1 (BONMASSAR GIORGIO [US] ET AL) 9 April 2015 (2015-04-09) <br> * paragraph [0048] * | 5 | |
| | ----- | | |
| Y | US 2007/255149 A1 (DECAMPLI WILLIAM M [US]) 1 November 2007 (2007-11-01) <br> * paragraph [0057] * <br> * figures 3A,3B * | 8,9 | |
| | ----- <br> -/-- | | |

**TECHNICAL FIELDS SEARCHED (IPC)**

A61B
A61F

## INCOMPLETE SEARCH

The Search Division considers that the present application, or one or more of its claims, does/do not comply with the EPC so that only a partial search (R.62a, 63) has been carried out.

Claims searched completely :

Claims searched incompletely :

Claims not searched :

Reason for the limitation of the search:

**see sheet C**

| Place of search | Date of completion of the search | Examiner |
|---|---|---|
| Munich | 12 January 2023 | Ebbinghaus, M |

EPO FORM 1503 03.82 (P04E07)

Europäisches
Patentamt

European
Patent Office

Office européen
des brevets

**PARTIAL EUROPEAN SEARCH REPORT**

Application Number

**EP 22 18 0903**

## DOCUMENTS CONSIDERED TO BE RELEVANT

| Category | Citation of document with indication, where appropriate, of relevant passages | Relevant to claim |
|---|---|---|
| Y | WO 2022/133081 A1 (MOVANO INC [US]) 23 June 2022 (2022-06-23) * claims 110,117,118 * * figure 21 * ----- | 11,12 |
| A | Watches Tv: "Watchmaking: A Life Saving Machine You Didn't Expect!", , 22 February 2022 (2022-02-22), XP093013253, Retrieved from the Internet: URL:https://www.youtube.com/watch?v=Ck3ukKIkbiA [retrieved on 2023-01-12] * the whole document * ----- | 1-12 |
| A | CN 102 166 126 A (MAOZHOU TIAN) 31 August 2011 (2011-08-31) * the whole document * ----- | 1 |
| A | SEKARSKI N ET AL: "Doppler-guided regulation of a telemetrically operated adjustable pulmonary banding system", JOURNAL OF THE AMERICAN COLLEGE OF CARDIOLOGY, ELSEVIER, AMSTERDAM, NL, vol. 44, no. 5, 1 September 2004 (2004-09-01), pages 1087-1094, XP004547179, ISSN: 0735-1097, DOI: 10.1016/J.JACC.2004.05.061 * the whole document * ----- | 1 |

CLASSIFICATION OF THE APPLICATION (IPC)

TECHNICAL FIELDS SEARCHED (IPC)

EPO FORM 1503 03.82 (P04C10)

page 2 of 2

Europäisches
Patentamt

European
Patent Office

Office européen
des brevets

**INCOMPLETE SEARCH
SHEET C**

Application Number

EP 22 18 0903

Claim(s) completely searchable:
        1-12

Claim(s) not searched:
        13-15

Reason for the limitation of the search (non-patentable invention(s)):

The subject-matter of claims 13 to 15 is excluded from Art. 53(a) EPC because the commercial exploitation of the inventions defined in these claims would be contrary to morality.
In the present case, the animal model is not genetically modified and only Article 53(a) EPC must be considered (but not Rule 28 EPC). However, T315/03 defined steps of the Rule 28(1)(d) EPC balancing test that must be applied to the stricter 'real' Article 53(a) EPC test (both are considered in T 315/03).
The non-human animal model of the objected claims is likely to suffer. This is evident from the triggering of abnormal pressure in the body vessel and cardiac failure. However, the corresponding likely substantial medical benefit (or benefit to mankind under Article 53(a) EPC according to T19/90) must be established over the whole scope of the claims, i.e. for any animal falling within the scope of the claim (see Guidelines G-II.5.3(iv)). This must be supported by evidence and the mere argument of providing different animal models is not considered sufficient (see T 315/03 Reason 12.2.3). The evidence must be in the form that each animal is specifically suited as a model of cardiac failure (more than hypothetical). This can be in the form of a scientific publication (e.g. examples of pigs used as model of cardiac failure and the advantages in view of their similitude with human). In the present case, the contribution is entirely hypothetical and no model has been produced.
Hence, claims 13 to 15 are objected under Art. 53(a) EPC.
In accordance with Rule 63(1) EPC (see also Guidelines G-II.4.1), the applicant has been invited to indicate the subject-matter to be searched in view of the above findings with respect to claims 13 to 15. In reply, the applicant has filed an amended set of claims identical to the original claims set with claims 13 to 15 deleted. While no amendments can be filed at this stage of the procedure (Rule 137(1) EPC), the applicant's submission is understood as an indication to limit the search to claims 1-12 of the original claims set. The present search report is therefore limited to these claims (Rule 63(2) EPC).

## ANNEX TO THE EUROPEAN SEARCH REPORT
## ON EUROPEAN PATENT APPLICATION NO.

EP 22 18 0903

This annex lists the patent family members relating to the patent documents cited in the above-mentioned European search report.
The members are as contained in the European Patent Office EDP file on
The European Patent Office is in no way liable for these particulars which are merely given for the purpose of information.

12-01-2023

| Patent document cited in search report | | Publication date | Patent family member(s) | | Publication date |
|---|---|---|---|---|---|
| US 2010125288 | A1 | 20-05-2010 | NONE | | |
| US 2020187816 | A1 | 18-06-2020 | US | 2020187815 A1 | 18-06-2020 |
| | | | US | 2020187816 A1 | 18-06-2020 |
| | | | US | 2020187817 A1 | 18-06-2020 |
| | | | US | 2020187818 A1 | 18-06-2020 |
| | | | US | 2020187819 A1 | 18-06-2020 |
| | | | US | 2020187820 A1 | 18-06-2020 |
| | | | US | 2020187867 A1 | 18-06-2020 |
| | | | US | 2020191909 A1 | 18-06-2020 |
| | | | US | 2020192426 A1 | 18-06-2020 |
| | | | US | 2020192427 A1 | 18-06-2020 |
| | | | US | 2020192428 A1 | 18-06-2020 |
| | | | US | 2020195293 A1 | 18-06-2020 |
| US 2015099959 | A1 | 09-04-2015 | US | 2015099959 A1 | 09-04-2015 |
| | | | WO | 2013163503 A1 | 31-10-2013 |
| US 2007255149 | A1 | 01-11-2007 | NONE | | |
| WO 2022133081 | A1 | 23-06-2022 | NONE | | |
| CN 102166126 | A | 31-08-2011 | NONE | | |

EPO FORM P0459

For more details about this annex : see Official Journal of the European Patent Office, No. 12/82

**REFERENCES CITED IN THE DESCRIPTION**

**Patent documents cited in the description**

- EP 1072282 A